(19) Europäisches Patentamt
European Patent Office
Office européen des brevets

(11) Veröffentlichungsnummer : 0 057 367
B1

(12) # EUROPÄISCHE PATENTSCHRIFT

(45) Veröffentlichungstag der Patentschrift : **28.12.83**

(51) Int. Cl.³ : **C 07 D213/64**, A 01 N 43/40

(21) Anmeldenummer : **82100318.3**

(22) Anmeldetag : **18.01.82**

(54) **2-Pyridyloxyacetanilid-Verbindungen, Verfahren zu ihrer Herstellung und ihre Verwendung.**

(30) Priorität : **28.01.81 JP 10110/81**

(43) Veröffentlichungstag der Anmeldung : **11.08.82 Patentblatt 82/32**

(45) Bekanntmachung des Hinweises auf die Patenterteilung : **28.12.83 Patentblatt 83/52**

(84) Benannte Vertragsstaaten : **AT BE CH DE FR GB IT LI NL SE**

(56) Entgegenhaltungen :
**CH-A- 625 940**
**DE-A- 2 637 886**
**GB-A- 1 472 485**
**US-A- 4 110 104**

(73) Patentinhaber : **NIHON TOKUSHU NOYAKU SEIZO K.K.**
**No.8, 2-chome, Nihonbashi Muromachi, Chuo-Ku Tokyo (JP)**

(72) Erfinder : **Aya, Masahiro**
**2-844, Suzuki-cho**
**Kodaira-shi Tokyo (JP)**
Erfinder : **Saito, Junichi**
**3-7-12, Osawa**
**Mitaka-shi Tokyo (JP)**
Erfinder : **Yasui, Kazuomi**
**7-6-15 Shakuji-dai**
**Nerima-ku Tokyo (JP)**
Erfinder : **Kakabu, Shinzo**
**47-15, Oya-cho**
**Hachioji-shi Tokyo (JP)**
Erfinder : **Kamochi, Atsumi**
**2-24-10, Toyoda**
**Hino-shi Tokyo (JP)**
Erfinder : **Yamaguchi, Naoko**
**3064-1, Hino-shi**
**Tokyo (JP)**

(74) Vertreter : **Gremm, Joachim, Dr. et al**
**Bayer AG c/o Zentralbereich Patente, Marken und Lizenzen**
**D-5090 Leverkusen 1, Bayerwerk (DE)**

**EP 0 057 367 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

2-Pyridyloxyacetanilid-Verbindungen, Verfahren zu ihrer Herstellung und ihre Verwendung

Die Erfindung betrifft 2-Pyridyloxyacetanilid-Verbindungen, Verfahren zur Herstellung dieser Verbindungen sowie diese Verbindungen als wirksame Inhaltsstoffe enthaltende Herbizide.

Sie betrifft die durch die allgemeine Formel (I) dargestellten 2-Pyridyloxyacetanilid-Verbindungen

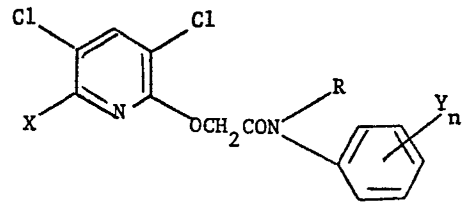

(I)

in der X für ein Wasserstoff-Atom, ein Halogen-Atom, eine $C_1$- bis $C_4$-Alkyl-Gruppe oder eine $C_1$- bis $C_4$-Halogenalkyl-Gruppe, R für eine $C_1$- bis $C_4$-Alkyl-Gruppe, Y für ein Halogen-Atom, eine $C_1$- bis $C_4$-Alkyl-Gruppe, eine $C_1$- bis $C_4$-Alkoxy-Gruppe oder eine $C_1$- bis $C_4$-Halogenalkyl-Gruppe und n für 0, 1, 2 oder 3 steht, sowie Verfahren zu deren Herstellung und deren Verwendung als Herbizide.

Die neuen Verbindungen der allgemeinen Formel (I) können beispielsweise durch die folgenden Verfahren hergestellt werden :

Die Verfahren zur Herstellung der Verbindungen der allgemeinen Formel (I) sind dadurch gekennzeichnet, daß ein 2-Chloropyridin der allgemeinen Formel (II)

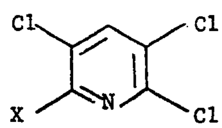

(II)

in der X die vorstehend genannte Bedeutung hat, mit einem Glykolsäure-N-alkylanilid der allgemeinen Formel (III)

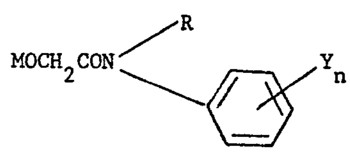

(III)

in der R, Y und n die vorstehend genannten Bedeutungen haben und M für ein Wasserstoff-Atom oder ein Alkalimetall-Atom steht, wahlweise in Gegenwart eines säurebindenden Mittels, umgesetzt wird oder ein 2-Pyridinol-Natriumsalz der allgemeinen Formel (IV)

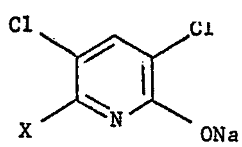

(IV)

in der X die vorstehend genannte Bedeutung hat, mit einem Chloroaceto-N-alkylanilid der allgemeinen Formel (V)

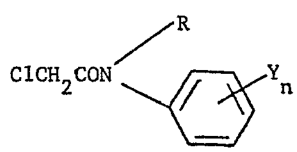

(V)

in der R, Y und n die vorstehend genannten Bedeutungen haben, umgesetzt wird.

Die Erfindung betrifft außerdem ein Herbizid, das ein 2-Pyridyloxyacetanilid der allgemeinen Formel (I) als wirksamen Inhaltsstoff enthält.

Aus der GB-PS 1 472 485 ist bekannt, daß 6-Fluoro-3,5-dihalogeno-2-pyridyloxamid-Verbindungen der allgemeinen Formel (VI)

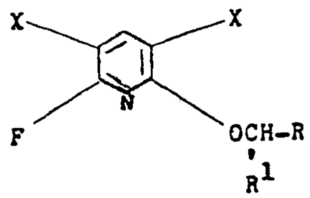

(VI)

in der X für Chlor, Brom oder Iod, $R^1$ für ein Wasserstoff-Atom oder eine Methyl-Gruppe und R für die (nicht eingezeichnete) Gruppe $CONR^4R^4$, in der $R^4$ ein Wasserstoff-Atom oder eine Alkyl-Gruppe mit 1-8 Kohlenstoff-Atomen bezeichnet, steht, herbizide Wirkung aufweisen.

Eingehende Untersuchungen der Anmelderin haben zu dem Ergebnis geführt, daß die 2-Pyridyloxyanilid-Verbindungen der allgemeinen Formel (I), die bisher in der Literatur nicht beschrieben wurden, eine ausgezeichnete herbizide Wirksamkeit besitzen und, wie durch die Ergebnisse der weiter unten beschriebenen Vergleichstests dargelegt wird, herausragende herbizide Wirkungen zeigen, die aufgrund der Kenntnis anderer, analoger Verbindungen in keiner Weise vorhersehbar waren.

Gegenstand der Erfindung ist demnach, die Verbindungen der allgemeinen Formel (I), ein Verfahren zu deren Herstellung sowie deren Verwendung als Herbizide verfügbar zu machen.

Wie aus der allgemeinen Formel (I) ersichtlich ist, sind die Verbindungen gemäß der Erfindung dadurch gekennzeichnet, daß sie ein N-Alkylanilid der 2-Pyridyloxyessigsäure als Skelett besitzen.

Die Verbindungen gemäß der Erfindung können günstig als Herbizide zur Unkrautbekämpfung verwendet werden, da sie eine geringe Toxizität für Warmblüter und eine gute Selektivität in Bezug auf die angebauten Pflanzen besitzen, d. h., daß sie die Eigenschaft haben, bei den üblicherweise angewandten Konzentrationen keine Phytotoxizität gegenüber Kulturpflanzen aufzuweisen. Das Herbizid gemäß der Erfindung zeigt eine ausgezeichnete selektive Bekämpfungswirksamkeit, insbesondere bei Verwendung als Mittel für die Bodenbehandlung vor dem Auflaufen der Reisfeld-Unkräuter und als Behandlungsmittel für den Boden sowie für die Stengel und Blätter von Reisfeld-Unkräutern. Wie vorstehend festgestellt, sind die Verbindungen gemäß der Erfindung ausgezeichnet hinsichtlich ihrer Sicherheit, zeigen hervorragende herbizide Wirksamkeit und weisen ein breites herbizides Spektrum auf.

Sie sind dadurch gekennzeichnet, daß sie eine bemerkenswerte herbizide Wirkung gegenüber den nachstehend beispielhaft genannten Reisfeld-Unkräutern entfalten, ohne Phytotoxizität gegenüber den im Wasser aufwachsenden Reispflanzen zu zeigen.

Pflanzenname :
Wissenschaftliche lateinische Bezeichnung

Dikotyledonen :
Rotala indica Koehne
Lindernia Procumbens Philcox
Ludwiga prostrata Roxburgh
Potamogeton distinctus A. Bennett
Elatine triandra Schkuhr

Monokotyledonen :
Echinochloa crus-galli Beauvois var.
Monochoria vaginalis Presl.
Eleocharis acicularis L.
Eleocharis Kuroguwai Ohwi
Cyperus difformis L.
Cyperus serotinus Rottboell
Sagittaria pygmaea Miq
Alisma canaliculatum A. Br. et Bouche
Scirpus juncoides Roxburgh var.

Sie sind weiterhin dadurch gekennzeichnet, daß sie eine bemerkenswerte herbizide Wirkung gegenüber den nachstehend beispielhaft genannten Unkräutern auf landwirtschaftlichen Nutzflächen entfalten ohne Phytotoxizität gegenüber den folgenden Feldfrüchten zu zeigen.

Pflanzenname :
Wissenschaftliche lateinische Bezeichnung

Dikotyledonen :
Polygonum sp.
Chenopodium album Linnaeus
Stellaria media Villars
Portulaca oleracea Linnaeus

Monokotyledonen :
Echinochloa crus-galli Beauv. var.
Digitaria adscendens Henr.
Cyperus iria L.

Für die Feldfrüchte seien nachstehende Beispiele angegeben : Als Beispiele für Dikotyledonen seien Senf, Baumwolle, Möhren, Bohnen, Kartoffeln, Rüben und Kohl erwähnt. Als Beispiele für Monokotyledonen seien Mais, Reis, Roggen, Weizen, Gerste, Hirse und Zuckerrohr erwähnt.

Die oben angegebenen Pflanzenarten sind als typische Beispiele der durch die lateinischen Namen bezeichneten Gattungen zu verstehen.

Die Anwendbarkeit der wirksamen Verbindungen gemäß der Erfindung ist nicht auf die Unkräuter der Reisanpflanzungen und landwirtschaftlichen Nutzflächen beschränkt, sondern sie sind auch wirksam gegen solche Unkräuter wie Binsen, oder gegen Unkräuter auf Brachland. Der Begriff « Unkräuter », wie er hierin verwendet wird, bezeichnet im weitesten Sinne sämtliche Pflanzen, die an Stellen wachsen, wo sie nicht erwünscht sind.

Die 2-Pyridyloxyacetanilid-Verbindungen der allgemeinen Formel (I) gemäß der Erfindung können beispielsweise mittels der nachstehenden Verfahren A) und B) synthetisiert werden.

Verfahren A) :

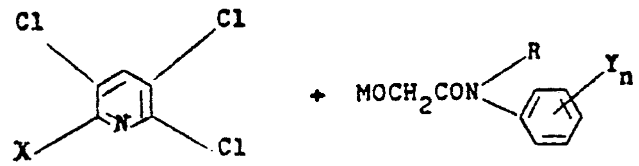

(II) (III)

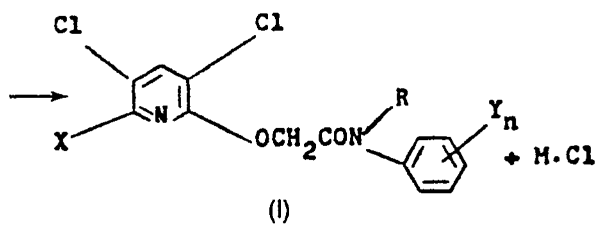

(I)

(in den Formeln haben X, R, Y, n und M die vorstehend genannte Bedeutung).

Bei dem Verfahren zur Herstellung der Verbindung der allgemeinen Formel (I), das durch das vorstehende Reaktionsschema wiedergegeben wird, bezeichnet X ein Wasserstoff-Atom, ein Halogen-Atom, wie Chlor, Brom, Iod und Fluor, eine $C_1$-$C_4$-Alkyl-Gruppe, wie Methyl, Ethyl, n-(oder iso-)Propyl und n-(iso-, sec.- oder tert.-)Butyl, oder eine $C_1$-$C_4$-Halogenalkyl-Gruppe, wie Chloro-(Dichloro- oder Trichloro-)methyl und Fluoro-(Difluoro- oder Trifluoro-)methyl.

Als Reste R seien dieselben niederen Alkyl-Gruppen genannt, die vorstehend beispielhaft erwähnt wurden.

Y kann für die gleichen Halogen-Atome, niederen Alkyl-Gruppen und Halogenalkyl-Gruppen wie oben angegeben, sowie für $C_1$-$C_4$-Alkoxy-Gruppen, wie Methoxy, Ethoxy, n-(oder iso-)Propoxy und n-(iso-, sec.- oder tert.-)Butoxy, stehen.

Beispiele für die durch die allgemeine Formel (II) wiedergegebenen 2-Chloropyridine, die als Ausgangsmaterial eingesetzt werden, sind die folgenden Verbindungen :

2,3,5-Trichloropyridin,
2,3,5,6-Tetrachloropyridin,
6-Methyl-2,3,5-trichloropyridin,
6-Ethyl-2,3,5-trichloropyridin,
6-n-(oder iso-)Propyl-2,3,5-trichloropyridin,
6-n-(iso-, sec.- oder tert.-)Butyl-2,3,5-trichloropyridin,
6-Chloromethyl-(Dichloromethyl- oder Trichloromethyl-)-2,3,5-trichloropyridin und
6-Fluoromethyl-(Difluoromethyl- oder Trifluoromethyl-)-2,3,5-trichloropyridin.

Beispiele für die Glykolsäure-N-alkylanilide der allgemeinen Formel (III), das andere Ausgangsmaterial, sind die folgenden Verbindungen :

Glykolsäure-N-methyl-[ethyl-, propyl-(oder iso-propyl), oder butyl-(iso, sec.- oder tert.-butyl)]-anilid,
Glykolsäure-N-methyl-2-(3- oder 4-)chloroanilid,
Glykolsäure-N-methyl-2-(3- oder 4-)bromoanilid,

Glykolsäure-n-methyl-2-(3- oder 4-)fluoroanilid,
Glykolsäure-N-methyl-2-(3- oder 4-)methylanilid,
Glykolsäure-N-methyl-2-(3- oder 4-)ethylanilid,
Glykolsäure-N-methyl-2-(3- oder 4-)propyl-(oder iso-propyl-)anilid,
Glykolsäure-N-methyl-2-(3- oder 4-)butyl-(iso-, sec.- oder tert.-butyl-)anilid,
Glykolsäure-N-methyl-2-(3- oder 4-)methoxyanilid,
Glykolsäure-N-methyl-2-(3- oder 4-)ethoxyanilid,
Glykolsäure-N-methyl-2-(3- oder 4-)propoxy-(oder iso-propoxy-)anilid,
Glykolsäure-N-methyl-2-(3- oder 4-)butoxy-(iso-, sec.- oder tert.-butoxy-)anilid,
Glykolsäure-N-methyl-2-(3- oder 4-)chloromethyl-(dichloromethyl- oder trichloromethyl-)anilid,
Glykolsäure-N-methyl-2-(3- oder 4-)fluoromethyl-(difluoromethyl- oder trifluoromethyl-)anilid,
Glykolsäure-N-methyl-2,4-(2,3-, 2,6-, 3,4- oder 3,5-)dichloroanilid,
Glykolsäure-N-methyl-2,3-(2,4-, 3,4- oder 3,5-)dimethylanilid und
Glykolsäure-N-methyl-2,4,5-(oder 2,4,6-)trichloroanilid.

Eine Ausführungsform des vorgenannten Verfahrens wird mit Hilfe eines typischen Beispiels erläutert :

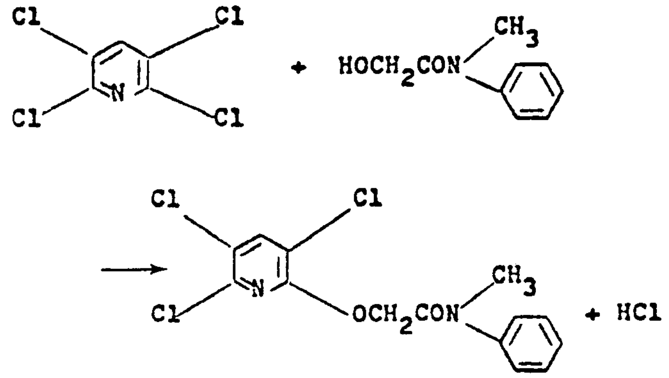

Das Verfahren A) zur Herstellung der Verbindungen gemäß der Erfindung wird zweckmäßig in einem Lösungsmittel oder Verdünnungsmittel durchgeführt. Für diesen Zweck können alle inerten Lösungsmittel und Verdünnungsmittel verwendet werden.

Beispiele für derartige Lösungs- oder Verdünnungsmittel umfassen Wasser, aliphatische, alicyclische und aromatische Kohlenwasserstoffe (ggf. chlorierte) wie Hexan, Cyclohexan, Petroläther, Ligroin, Benzol, Toluol, Xylol, Methylenchlorid, Chloroform, Kohlenstofftetrachlorid, Ethylenchlorid, Trichlorethylen und Chlorbenzol, Äther wie Diethyläther, Methyl-ethyläther, Di-isopropyläther, Dibutyläther, Propylenoxid, Dioxan und Tetrahydrofuran, Ketone wie Aceton, Methyl-ethylketon, Methyl-isopropylketon und Methyl-isobutylketon, Nitrile wie Acetonitril, Propionitril und Acrylnitril, Alkohole wie Methanol, Ethanol, Isopropanol, Butanol und Ethylenglykol, Ester wie Ethylacetat und Amylacetat, Säureamide wie Dimethylformamid und Dimethylacetamid, Sulfone und Sulfoxide wie Dimethylsulfoxid und Sulfolan, und Basen wie Pyridin.

Die Reaktion gemäß der Erfindung kann in Gegenwart eines säurebindenden Mittels ausgeführt werden, wie bereits oben erwähnt wurde. Beispiele solcher säurebindenden Mittel sind Alkalimetall-Hydroxide, -Carbonate, -Bicarbonate und -Alkoholate, und tertiäre Amine wie Triethylamin, Trimethylamin und Pyridin, die im allgemeinen verwendet werden.

Das Verfahren A) gemäß der Erfindung kann innerhalb eines weiten Temperaturbereichs durchgeführt werden. Im allgemeinen wird es bei einer Temperatur zwischen – 20 °C und dem Siedepunkt der Mischung, vorzugsweise bei einer Temperatur zwischen 0 und 100 °C durchgeführt. Zweckmäßigerweise wird die Reaktion bei atmosphärischem Druck durchgeführt, jedoch kann auch unter erhöhtem oder vermindertem Druck gearbeitet werden.

Verfahren B) :

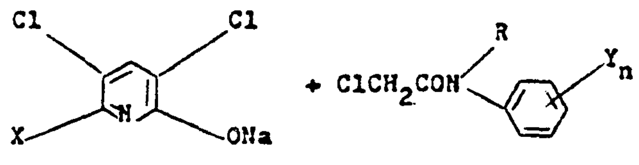

(IV) (V)

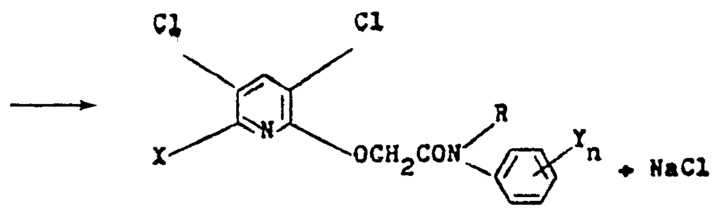

(I)

(In den Formeln haben X, R, Y und n die vorstehend genannten Bedeutungen.)

Bei dem Verfahren zur Herstellung der Verbindung der allgemeinen Formel (I), das durch das vorstehende Reaktionsschema veranschaulicht wird, sind Beispiele für das 2-Pyridinolnatriumsalz der allgemeinen Formel (IV) als eines der Ausgangsmaterialien die folgenden Verbindungen :

Natrium-3,5-dichloro-2-pyridinolat,
Natrium-3,5,6-trichloro-2-pyridinolat,
Natrium-6-fluoro-3,5-dichloro-2-pyridinolat,
Natrium-6-methyl-3,5-dichloro-2-pyridinolat,
Natrium-6-ethyl-3,5-dichloro-2-pyridinolat,
Natrium-6-n-(oder iso-)propyl-3,5-dichloro-2-pyridinolat,
Natrium-6-n-(iso-, sec.- oder tert.-)butyl-3,5-dichloro-2-pyridinolat,
Natrium-6-chloromethyl-(dichloromethyl- oder trichloromethyl-)-3,5-dichloro-2-pyridinolat, und
Natrium-6-fluoromethyl-(difluoromethyl- oder trifluoromethyl-)-3,5-dichloro-2-pyridinolat.

Beispiele für die Chloroaceto-N-alkylanilide der allgemeinen Formel (V), das andere Ausgangsmaterial, sind die folgenden Verbindungen :

Chloroaceto-N-methyl-[ethyl-, propyl-(oder iso-propyl) oder butyl-(iso, sec.-, oder tert.-butyl)]-anilid,
Chloroaceto-N-methyl-2-(3- oder 4-)chloroanilid,
Chloroaceto-N-methyl-2-(3- oder 4-)bromoanilid,
Chloroaceto-N-methyl-2-(3- oder 4-)fluoroanilid,
Chloroaceto-N-methyl-2-(3- oder 4-)methylanilid,
Chloroaceto-N-methyl-2-(3- oder 4-)ethylanilid,
Chloroaceto-N-methyl-2-(3- oder 4-)propyl-(oder iso-propyl-)anilid,
Chloroaceto-N-methyl-2-(3- oder 4-)butyl-(iso-, sec.- oder tert.-butyl-)anilid,
Chloroaceto-N-methyl-2-(3- oder 4-)methoxyanilid,
Chloroaceto-N-methyl-2-(3- oder 4-)ethoxyanilid,
Chloroaceto-N-methyl-2-(3- oder 4-)propoxy-(oder iso-propoxy)anilid,
Chloroaceto-N-methyl-2-(3- oder 4-)butoxy-(iso-, sec.- oder tert.-butoxy-)anilid,
Chloroaceto-N-methyl-2-(3- oder 4-)chloromethyl-(dichloromethyl- oder trichloromethyl-)anilid,
Chloroaceto-N-methyl-2-(3- oder 4-)fluoromethyl-(difluoromethyl- oder trifluoromethyl-)anilid,
Chloroaceto-N-methyl-2,4-(2,3-, 2,6-, 3,4- oder 3,5-)dichloroanilid,
Chloroaceto-N-methyl-2,3-(2,4-, 3,4- oder 3,5-)dimethyl-anilid, und
Chloroaceto-N-methyl-2,4,5-(oder 2,4,6-)trichloroanilid.

Anhand eines typischen Beispiels wird eine Ausführungsform des vorgenannten Verfahrens besonders erläutert :

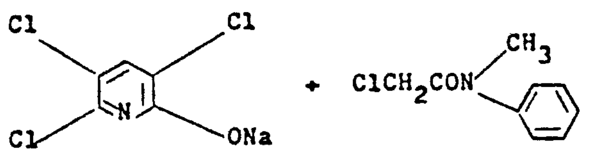

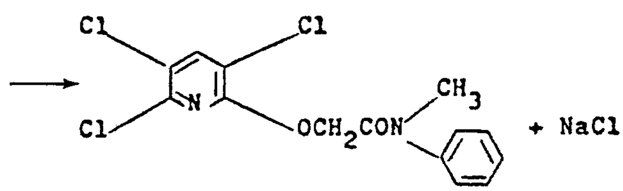

Für die Durchführung des vorbeschriebenen Verfahrens können die gleichen inerten Lösungsmittel oder Verdünnungsmittel zweckmäßigerweise verwendet werden, die weiter oben beispielhaft genannt wurden.

Das Verfahren B) gemäß der Erfindung kann innerhalb eines weiten Temperaturbereichs durchgeführt werden. Im allgemeinen kann es bei einer Temperatur zwischen – 20 °C und dem Siedepunkt der Mischung, zweckmäßigerweise bei einer Temperatur zwischen etwa 0 und etwa 100 °C, durchgeführt werden. Vorzugsweise wird die Reaktion unter normalem atmosphärischen Druck durchgeführt, aber es kann auch unter erhöhtem oder vermindertem Druck gearbeitet werden.

Typische Beispiele der Verbindungen gemäß der Erfindung, die mit Hilfe der Verfahren A) und B) hergestellt werden können, sind nachstehend aufgeführt :

3,5-Dichloro-2-pyridyloxyaceto-N-methylanilid,
3,5-Dichloro-2-pyridyloxyaceto-N-methyl-2-(3- oder 4-)chloroanilid,
3,5,6-Trichloro-2-pyridyloxyaceto-N-methylanilid,
3,5-Dichloro-6-methyl-2-pyridyloxyaceto-N-methylanilid,
3,5,6-Trichloro-2-pyridyloxyaceto-N-methyl-2-(3- oder 4-)chloroanilid,
3,5,6-Trichloro-2-pyridyloxyaceto-N-methyl-2-(3- oder 4-)bromoanilid,
3,5,6-Trichloro-2-pyridyloxyaceto-N-methyl-2-(3- oder 4-)fluoroanilid,
3,5,6-Trichloro-2-pyridyloxyaceto-N-methyl-2-(3- oder 4)methylanilid,
3,5,6-Trichloro-2-pyridyloxyaceto-N-methyl-2-(3- oder 4-)ethylanilid,
3,5,6-Trichloro-2-pyridyloxyaceto-N-methyl-2-(3- oder 4-)propylanilid,
3,5,6-Trichloro-2-pyridyloxyaceto-N-methyl-2-(3- oder 4-)iso-propylanilid,
3,5,6-Trichloro-2-pyridyloxyaceto-N-methyl-2-(3- oder 4-)methoxyanilid,
3,5,6-Trichloro-2-pyridyloxyaceto-N-methyl-2-(3- oder 4-)ethoxyanilid,
3,5,6-Trichloro-2-pyridyloxyaceto-N-methyl-2-(3- oder 4-)iso-propoxyanilid,
3,5,6-Trichloro-2-pyridyloxyaceto-N-methyl-2-(3- oder 4-)trifluoromethylanilid,
3,5,6-Trichloro-2-pyridyloxyaceto-N-methyl-2,4-(oder 2,6-)dichloroanilid,
3,5,6-Trichloro-2-pyridyloxyaceto-N-methyl-2,4,5-(oder 2,4,6-)trichloroanilid
3,5,6-Trichloro-2-pyridyloxyaceto-N-methyl-2,3-dimethylanilid,
3,5,6-Trichloro-2-pyridyloxyaceto-N-methyl-2,4-dimethylanilid,
3,5-Dichloro-2-pyridyloxyaceto-N-ethylanilid,
3,5-Dichloro-2-pyridyloxyaceto-N-propylanilid,
3,5-Dichloro-6-fluoro-2-pyridyloxyaceto-N-methylanilid,
3,5-Dichloro-6-trifluoromethyl-2-pyridyloxyaceto-N-methylanilid,
und
3,5-Dichloro-6-trichloromethyl-2-pyridyloxyaceto-N-methylanilid.

Zur Anwendung einer Verbindung gemäß der Erfindung als Herbizid kann diese unmittelbar nach dem Verdünnen mit Wasser verwendet werden, oder sie kann dazu mit Hilfe der in der landwirtschaftchemischen Produktion allgemein angewandten Verfahren und unter Verwendung landwirtschaftlich unbedenklicher Hilfsstoffe in eines der verschiedenen gebräuchlichen Präparate überführt werden. Beim praktischen Gebrauch können diese verschiedenen Präparate entweder unmittelbar oder nach dem Verdünnen mit Wasser auf die gewünschte Konzentration zur Anwendung gebracht werden.

Zu den landwirtschaftlich unbedenklichen Hilfsstoffen gehören beispielsweise Verdünnungsmittel (Lösungsmittel, Streckmittel, Träger), oberflächenaktive Mittel (Lösungsvermittler, Emulgatoren, Dispersionsmittel, Netzmittel), Stabilisatoren, Haftmittel und Aerosol-Treibgase.

Beispiele für Lösungsmittel sind Wasser sowie organische Lösungsmittel, beispielsweise Kohlenwasserstoffe [wie n-Hexan, Petroläther, Naphtha, Mineralölfraktionen, (Paraffin-Wachse, Kerosin, Leichtöl, Mittelöl, Schweröl), Benzol, Toluol und Xylol], halogenierte Kohlenwasserstoffe (wie Methylenchlorid, Kohlenstofftetrachlorid, Trichlorethylen, Ethylenchlorid, Ethylendibromid, Chlorbenzol und Chloroform), Alkohole (wie Methylalkohol, Ethylalkohol, Propylalkohol und Ethylenglykol), Äther (wie Ethyläther, Ethylenoxid und Dioxan), Alkohol-Äther (wie Ethylenglykol und Monomethyläther), Ketone (wie Aceton und Isophoron), Ester (wie Ethylacetat und Amylacetat), Amide (wie Dimethylformamid und Dimethylacetamid) und Sulfoxide (wie Dimethylsulfoxid).

Beispiele für Streckmittel oder Träger sind Pulver aus anorganischen Materialien wie Schwefel, gelöschter Kalk, Magnesiumkalk, Gips, Calciumcarbonat, Kieselerdegestein, Perlit, Bimsstein, Calcit, Diatomeenerde, amorphes Siliciumoxid, Aluminiumoxid, Zeolith und Tonmineralien (z. B. Tyrophyllit, Talk, Montmorillonit, Beidellit, Vermikulit, Kaolinit und Glimmer), Pulver aus pflanzlichem Material wie Getreidepulver, Stärke, verarbeitete Stärke, Zucker, Glucose und verkleinerte Pflanzenstiele und -stengel, sowie Pulver aus synthetischen Harzen wie Phenolharzen, Harnstoffharzen und Vinylchloridharzen.

Beispiele der oberflächenaktiven Mittel sind anionische oberflächenaktive Mittel, beispielsweise Alkylsulfat-Ester (wie Natriumlaurylsulfat), Arylsulfonsäuren (wie Alkylarylsulfonsäure-Salze, Natrium-Alkylnaphthalinsulfonat), Bernsteinsäure-Salze, und Polyethylenglykol-Alkylaryläther-Schwefelsäureester-Salze ; kationische oberflächenaktive Mittel, beispielsweise Alkylamine (wie Laurylamin, Stearyltrimethylammoniumchlorid und Alkyldimethyl-Benzylammoniumchlorid) und Polyoxyethylen-Alkylamine ; nichtionische oberflächenaktive Mittel, beispielsweise Polyoxyethylen-Glykoläther (wie Polyoxyethylen-Alkylaryläther und deren Kondensationsprodukte), Polyoxyethylen-Glykolester (wie Polyoxyethylen-Fettsäureester) und Ester mehrwertiger Alkohole (wie Polyoxyethylen-Sorbit-Monolaurat) ; sowie amphotere oberflächenaktive Mittel.

Andere Hilfsmittel sind beispielsweise Stabilisatoren, Haftmittel (wie landwirtschaftliche Seifen, Caseinkalk, Natriumalginat, Polyvinylalkohol (PVA), Haftmittel vom Vinylacetat-Typ und Haftmittel auf Acrylbasis), die Wirksamkeit verlängernde Mittel, Dispersionsstabilisatoren (wie Casein, Tragant, Carboxymethylcellulose (CMC), und Polyvinylalkohol (PVA)).

Die Verbindungen gemäß der Erfindung können mit Hilfe der allgemein bei der Herstellung von landwirtschaftlichen Chemikalien gebräuchlichen Verfahren zu verschiedenen Präparaten formuliert werden. Zu diesen Präparaten gehören beispielsweise emulgierbare Konzentrate, Öle, benetzbare Pulver, lösliche Pulver, Suspensionen, Stäube, Granalien, Mikrogranalien und Kapseln.

Das Herbizid gemäß der Erfindung kann den vorgenannten wirksamen Inhaltsstoff in einer Menge von etwa 0,01 bis etwa 100 Gewichtsprozent, vorzugsweise etwa 0,05 bis etwa 95 Gewichtsprozent enthalten.

Im praktischen Gebrauch beträgt der geeignete Gehalt des Wirkstoffs in den verschiedenen Präparaten und gebrauchsfertigen Präparaten im allgemeinen etwa 0,01 bis etwa 95 Gewichtsprozent, vorzugsweise etwa 0,05 bis etwa 60 Gewichtsprozent. Die Menge des aktiven Inhaltsstoffes kann je nach der Form des Präparats, dem Verfahren, dem Zweck, der Zeit und dem Ort seiner Anwendung, dem Zustand des Unkrautbefalls etc. geeignet variiert werden.

Falls erwünscht kann die Verbindung gemäß der Erfindung in Verbindung mit anderen landwirtschaftlichen Chemikalien zubereitet oder verwendet werden, beispielsweise mit Insektiziden, Fungiziden, Akariziden, Nematoziden, Viriziden, anderen Herbiziden, Pflanzen-Wachstumsregulatoren und Lockstoffen (wie Organophosphorester-Verbindungen, Carbamat-Verbindungen, Dithio-(oder- thiol-) carbamat-Verbindungen, Organochlor-Verbindungen, Dinitro-Verbindungen, organischen Schwefel- oder Metall-Verbindungen, Antibiotika, substituierten Diphenyläther-Verbindungen, Harnstoff-Verbindungen und Triazinverbindungen), und/oder Düngemitteln.

Durch die Einbeziehung anderer Wirkstoffe ist es möglich, ein breites herbizides Spektrum und eine genaue Bekämpfungswirkung zu erzielen, und infolge des Vermischens dieser Bestandteile ist ggf. auch ein synergistischer Effekt zu erwarten.

Die verschiedenen Zubereitungen oder gebrauchsfertigen Präparate, die einen Wirkstoff gemäß der Erfindung enthalten, können mit Hilfe der bei der Anwendung von Agrochemikalien allgemein gebräuchlichen Methoden zum Einsatz gebracht werden wie durch Versprühen (z. B. Spritzen, Nebeln, Zerstäuben, Stäuben, Streuen von Granalien, Wasseroberflächen-Anwendung oder Gießen), sowie Bodenbehandlung (z. B. Vermischen und Streuen). Sie können ebenfalls mit Hilfe des sogenannten ULV-Sprühverfahrens (ultralow volume spraying method) zum Einsatz gebracht werden, und bei diesem Verfahren kann der Wirkstoff in einer Konzentration von 100 % vorliegen.

Die Dosierung pro Einheitsfläche kann in geeigneter Weise variiert werden und beträgt beispielsweise etwa 0,1 bis etwa 3 kg/ha, vorzugsweise etwa 0,2 bis etwa 1 kg/ha, bezogen auf den Wirkstoff. In besonderen Fällen kann, und muß manchmal sogar, die Aufwandmenge den angegebenen Bereich über- oder unterschreiten.

Gemäß der Erfindung wird ein Herbizid verfügbar gemacht, das eine Verbindung der allgemeinen Formel (I) als Wirkstoff enthält, beispielsweise eine herbizide Mischung aus einer Verbindung der allgemeinen Formel (I) und einem Verdünnungsmittel (Lösungsmittel und/oder Streckmittel und/oder Träger) und/oder einem oberflächenaktiven Mittel und, falls weiterhin erforderlich, einem Stabilisator oder Haftmittel.

Gemäß der Erfindung wird auch ein Verfahren zur Unkrautbekämpfung verfügbar gemacht, das dadurch gekennzeichnet ist, daß man auf die Unkräuter und/oder ihren Lebensraum die Verbindung der allgemeinen Formel (I) allein oder in Verbindung mit einem Verdünnungsmittel (einem Lösungsmittel und/oder Streckmittel und/oder Träger) und/oder einem oberflächenaktiven Mittel und, falls weiterhin erforderlich, mit einem Stabilisator oder einem Haftmittel, aufbringt.

Die folgenden Beispiele erläutern mehrere Ausführungsformen der Erfindung näher.

### Beispiel 1 (benetzbares Pulver)

15 Teile der Verbindung Nr. 1 der Erfindung, 80 Teile einer 1 : 5-Mischung aus pulvriger Diatomeenerde und pulvrigem Ton, 2 Teile Natrium-Alkylbenzolsulfonat und 3 Teile Natrium-Alkylnaphthalinsulfonat/Formaldehyd-Kondensat werden pulverisiert und miteinander vermischt, wodurch ein benetzbares Pulver gebildet wird.

Das benetzbare Pulver wird mit Wasser verdünnt und tropfenweise auf Unkräuter und/oder deren Lebensraum aufgebracht.

### Beispiel 2 (emulgierbares Konzentrat)

30 Teile der Verbindung Nr. 2 der Erfindung, 55 Teile Xylol, 8 Teile Polyoxyethylen-Alkylphenyläther und 7 Teile Calcium-Alkylbenzolsulfonat werden unter Rühren miteinander vermischt, wodurch ein emulgierbares Konzentrat gebildet wird. Das emulgierbare Konzentrat wird mit Wasser verdünnt und tropfenweise auf Unkräuter und/oder deren Lebensraum aufgebracht.

Beispiel 3 (Staub)

2 Teile der Verbindung Nr. 3 der Erfindung und 98 Teile pulvriger Ton werden pulverisiert und miteinander vermischt, wobei ein Staub gebildet wird. Der Staub wird auf Unkräuter und/oder deren Lebensraum aufgestäubt.

Beispiel 4 (Staub)

Die Verbindung Nr. 4 der Erfindung (1,5 Teil), 0,5 Teil Isopropyl-Hydrogenphosphat (PAP) und 98 Teile pulvriger Ton werden pulverisiert und miteinander vermischt, wobei ein Staub gebildet wird. Der Staub wird auf Unkräuter und/oder deren Lebensraum aufgestäubt.

Beispiel 5 (Granulate)

Wasser (25 Teile) wird zu einer Mischung aus 10 Teilen der Verbindung Nr. 5 der Erfindung, 30 Teilen Bentonit (Montmorillonit), 58 Teilen Talkum und 2 Teilen Ligninsulfonat-Salz zugesetzt, und das Gemisch wird innig geknetet. Die Mischung wird mittels eines Extruder-Granulators zu Granalien mit einer Korngröße von 0,43 bis 2,0 mm (10 bis 40 mesh) verarbeitet und bei 40-50 °C getrocknet, wobei ein Granulat gebildet wird. Das Granulat wird auf Unkräuter und/oder deren Lebensraum aufgestreut.

Beispiel 6 (Granulate)

Ein Drehmischer wurde mit 95 Teilen Tonmineral-Teilchen mit einer Teilchengröße-Verteilung im Bereich von 0,2 bis 2 mm beschickt, und unter Drehen des Mischers werden 6,5 Teile der Verbindung Nr. 6 der Erfindung, aufgelöst in einem organischen Lösungsmittel, gleichmäßig auf die Tonmineral-Teilchen aufgesprüht. Die Teilchen werden dann bei 40-50 °C getrocknet, wodurch ein Granulat gebildet wird. Das Granulat wird auf Unkräuter und/oder deren Lebensraum aufgestreut.

Die Verbindungen gemäß der Erfindung haben sehr hohe Nützlichkeitswerte, weil sie im Vergleich zu Wirkstoffen mit analoger Struktur und Verbindungen von ähnlichem Wirksamkeitstypus, die bereits aus der Literatur bekannt sind, dadurch gekennzeichnet sind, daß ihre Wirksamkeiten wesentlich verbessert sind und daß sie eine sehr geringe Toxizität gegenüber Warmblütern aufweisen.

Die in unerwarteter Weise hervorragenden und ausgeprägten Wirkungen der Wirkstoffe gemäß der Erfindung können aus dem folgenden Ergebnis von Tests ersehen werden, in denen diese Verbindungen gegen verschiedene Unkräuter angewandt wurden.

Test-Beispiel 1

Test zur Behandlung des Bodens und von Stengeln und Blättern unter Bewässerungsbedingungen gegen im Wasser wachsende Reis-Unkräuter (Topf-Test) :

Herstellung einer Wirkstoff-Zubereitung

Träger : 5 Gewichtsteile Aceton,

Emulgator : 1 Gewichtsteil Benzyloxy-Polyglykoläther.

Eine Wirkstoffzubereitung in Form eines emulgierbaren Konzentrats wird durch Vermischen eines Teils des Wirkstoffs mit den vorbezeichneten Mengen Träger und Emulgator erhalten. Eine vorbestimmte Menge des Präparats wird durch Verdünnen mit Wasser erhalten.

Testverfahren

Wagner-Töpfe (2 $dm^2$) wurden mit Reis-Kulturboden gefüllt, und zwei Reis-Setzlinge (Varietät : Kinnampu) wurden in jeden Topf umgepflanzt. Samen von Echinochloa crus-galli, Cyperus iria L., Monochoria vaginalis Presl, Scirpus juncoides Roxburgh var. sowie von breitblättrigen Unkräutern, kleine Stücke von Eleocharis acicularis L. sowie Knollen von Cyperus serotinus Rottboell und Sagittaria pygmaea Miq. wurden in die Töpfe gegeben, und die Töpfe wurden in feuchtem Zustand gehalten. Als Echinochloa crus-galli etwa bis zum 2-Blattstadium gewachsen war (etwa 7-9 Tage nach der Einsaat), wurden die Töpfe mit Wasser bis zu einer Tiefe von etwa 6 cm gefüllt, und eine vorher festgelegte Menge der Verbindung gemäß der Erfindung wurde in Form einer Emulsion mittels einer Pipette zugegeben. Nach der Behandlung wurde das Wasser mit einer Geschwindigkeit von 2-3 cm pro Tag im Laufe von 2 Tagen aus den Töpfen heraussickern gelassen. Danach wurde die Wassertiefe in den Töpfen bei etwa 3 cm gehalten, und 4 Wochen nach der Behandlung mit der Chemikalie wurden die herbizide Wirkung und der Grad der Phytotoxizität auf einer Skala von 0-5 nach dem folgenden Maßstab bewertet.

Die Bewertung erfolgte auf der Basis der prozentualen Unkraut-Vernichtungsrate im Vergleich zu einer unbehandelten Fläche folgendermaßen :

Unkrautvernichtungsrate (bezogen auf die unbehandelte Fläche)

5 : mindestens 95 % (verdorrt)
4 : mindestens 80 %, jedoch weniger als 95 %
3 : mindestens 50 %, jedoch weniger als 80 %
2 : mindestens 30 %, jedoch weniger als 50 %
1 : mindestens 10 %, jedoch weniger als 30 %
0 : weniger als 10 % (unwirksam)

Die Phytotoxizität gegenüber Reispflanzen wurde im Vergleich zu der unbehandelten Fläche wie folgt bewertet:

Phytotoxizitätsrate im Vergleich mit der unbehandelten Fläche

5 : mindestens 90 % (Ausrottung)
4 : mindestens 50 %, jedoch weniger als 90 %
3 : mindestens 30 %, jedoch weniger als 50 %
2 : mindestens 10 %, jedoch weniger als 30 %
1 : mehr als 0, jedoch weniger als 10 %
0 : 0 % (keine Phytotoxizität)

Die Test-Ergebnisse sind in Tabelle I aufgeführt.

Tabelle 1

| Verbindung Nr. | Wirkstoffmenge kg/ha | Herbizide Wirkung | | | | | | | | Phytotoxizität gegenüber Reis |
|---|---|---|---|---|---|---|---|---|---|---|
| | | A | B | C | D | E | F | G | H | |
| 1 | 0,5 | 5 | 5 | 5 | 5 | 5 | 5 | 5 | 5 | 0 |
| 2 | 0,5 | 5 | 5 | 5 | 5 | 5 | 5 | 5 | 5 | 0 |
| 3 | 0,5 | 5 | 5 | 5 | 5 | 5 | 5 | 5 | 5 | 0 |
| 4 | 0,5 | 5 | 5 | 5 | 5 | 5 | 5 | 5 | 5 | 0 |
| 5 | 0,5 | 5 | 5 | 5 | 5 | 5 | 5 | 5 | 5 | 0 |
| 6 | 0,5 | 5 | 5 | 5 | 5 | 5 | 5 | 5 | 5 | 0 |
| 7 | 0,5 | 5 | 5 | 5 | 5 | 5 | 5 | 5 | 5 | 0 |
| 8 | 0,5 | 5 | 5 | 5 | 5 | 5 | 5 | 5 | 5 | 0 |
| 9 | 0,5 | 5 | 5 | 5 | 5 | 5 | 5 | 5 | 5 | 0 |
| 10 | 0,5 | 5 | 5 | 5 | 5 | 5 | 5 | 5 | 5 | 0 |
| 11 | 0,5 | 5 | 5 | 5 | 5 | 5 | 5 | 5 | 5 | 0 |
| 12 | 0,5 | 5 | 5 | 5 | 5 | 5 | 5 | 5 | 5 | 0 |
| 13 | 0,5 | 5 | 5 | 5 | 5 | 5 | 5 | 5 | 5 | 0 |
| 14 | 0,5 | 5 | 5 | 5 | 5 | 5 | 5 | 5 | 5 | 0 |
| 15 | 0,5 | 5 | 5 | 5 | 5 | 5 | 5 | 5 | 5 | 0 |
| 16 | 0,5 | 5 | 5 | 5 | 5 | 5 | 5 | 5 | 5 | 0 |
| 17 | 0,5 | 5 | 5 | 5 | 5 | 5 | 5 | 5 | 5 | 0 |
| 18 | 0,5 | 5 | 5 | 5 | 5 | 5 | 5 | 5 | 5 | 0 |
| 19 | 0,5 | 5 | 5 | 5 | 5 | 5 | 5 | 5 | 5 | 0 |
| Vergleichs-Verbindung | | | | | | | | | | |
| VI-1 | 0,5 | 2 | 1 | 4 | 2 | 3 | 4 | 2 | 3 | 0 |
| VI-2 | 0,5 | 1 | 1 | 4 | 1 | 1 | 3 | 1 | 1 | 0 |

Anmerkungen

i) Die die Verbindungen bezeichnenden Zahlen sind die gleichen wie die in den Synthese-Beispielen und der nachfolgenden Tabelle 2 verwendeten.

ii) In der Tabelle bezeichnen die Buchstaben A-H die folgenden Unkräuter:

A: Echinochloa crus-galli Beauv. var.,
B: Eleocharis acicularis L.,
C: Cyperus iria L.,
D: Scirpus juncoides Roxburgh var.,
E: Monochoria vaginalis Presl,
F: Breitblättrige Unkräuter (Lindernia Procumbens Philcox, Rotala indica Koehne, Elatine triandra Schk., etc.),
G: Cyperus serotinus Rottboell,
H: Sagittaria pygmaea Miq.

iii) Die Vergleichsverbindungen (vgl. GB-PS 1 472 485) waren die folgenden:

VI - 1 :

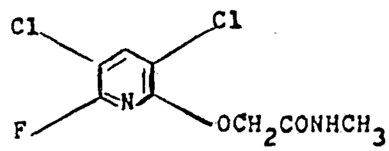

VI - 2 :

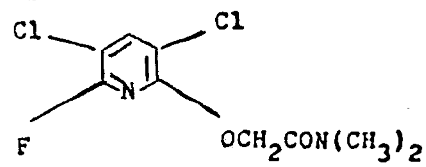

Test-Beispiel 2

Wuchshemmung bei Gerste

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

| Wirkstoff Nr. | Konzentration in % | Wuchshemmung in % |
|---|---|---|
| 1 | 0,05 | 40 |
| Kontrolle | - | = 0 |

Wirkstoff Nr. 1 =

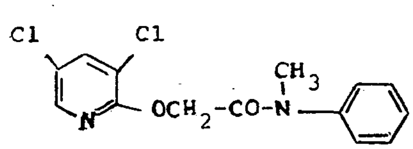

Die folgenden Synthese-Beispiele erläutern mehrere Beispiele der Verfahren zur Herstellung der Verbindungen gemäß der Erfindung.

Synthese-Beispiel 1

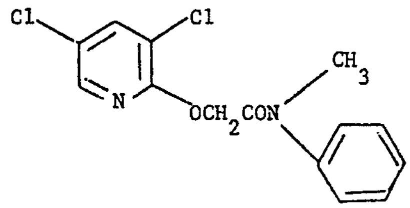

(Verbindung Nr. 1)

1,82 g 2,3,5-Trichloropyridin und 1,65 g Glykolsäure-N-methylanilid wurden in 30 ml Toluol aufgelöst, und 3,0 g Kaliumhydroxid und eine Spatelspitze Triethyl-Benzylammoniumchlorid wurden hinzugefügt. Die Mischung wurde 5 Stunden bei Raumtemperatur gerührt. Die Reaktionsmischung wurde in 50 ml Wasser gegossen, und die Toluol-Schicht wurde abgetrennt. Die alkalische Schicht wurde zweimal mit 20 ml Toluol gewaschen. Die Toluol-Schichten wurden vereinigt, mit 20 ml Wasser gewaschen und danach über wasserfreiem Natriumsulfat getrocknet. Das Trockenmittel wurde durch Filtration entfernt, und das Toluol wurde abdestilliert, wonach 2,13 g des gewünschten 3,5-Dichloro-2-pyridyloxyaceto-N-methylanilids (Ausbeute 68,5 %) mit dem Schmp. 146,5-148 °C erhalten wurden.

Synthese-Beispiel 2

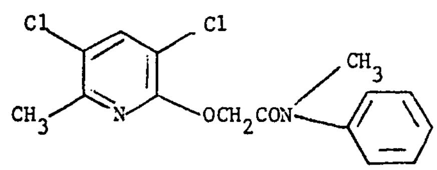

(Verbindung Nr. 2)

1,52 g Glykolsäure-N-methylanilid wurden zu einer Suspension von 1,02 g Kalium-tert.-butylat in 20 ml tert.-Butanol gegeben und 1,67 g 6-Methyl-2,3,5-trichloropyridin wurden hinzugefügt. Die Mischung wurde 12 Stunden bei 60 °C gerührt. Beim Eingießen der Reaktionsmischung in 70 ml Wasser fiel das gewünschte 3,5-Dichloro-6-methyl-2-pyridyloxyaceto-N-methylanilid aus. Die Kristalle wurden durch Filtration gesammelt und mit 5 ml kaltem Ethanol gewaschen. Die Menge betrug 2,50 g (Ausbeute 87,9 %) Schmp. 136-138 °C.

Synthese-Beispiel 3

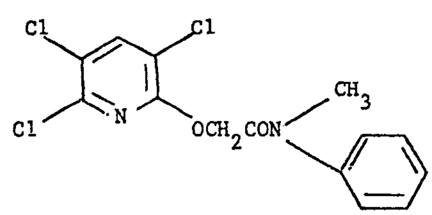

(Verbindung Nr. 3)

2 g Natrium-3,5,6-trichloro-2-pyridinolat und 0,035 g wasserfreies Natriumcarbonat wurden in 25 ml Dimethylsulfoxid aufgelöst, und die Lösung wurde auf 80 °C erhitzt. 1,54 g Chloroaceto-N-methylanilid wurden auf einmal hinzugegeben, und die Mischung wurde 3 Stunden bei 80 °C gerührt und anschließend in Eiswasser gegossen. Die ausgefallenen Kristalle wurden durch Filtration gesammelt, mit jeweils kleinen Mengen kaltem Äther und kaltem Hexan gewaschen und getrocknet, wonach 2,1 g 3,5-Dichloro-2-pyridyloxyaceto-N-methylanilid erhalten wurden. Ausbeute 75 %. Schmp. 140-142 °C.

Nach im wesentlichen dem gleichen Verfahren wie vorstehend beschrieben wurden die in Tabelle 2 aufgeführten Verbindungen erhalten.

Tabelle 2

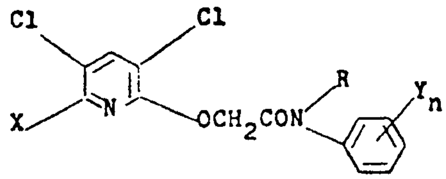

| Verbindung Nr. | X | R | $Y_n$ | Schmp., °C |
|---|---|---|---|---|
| 4 | H | $C_2H_5$ | - | 137 - 139 |
| 5 | H | $C_3H_7$ | - | 78 - 79 |
| 6 | H | $CH_3$ | 2-Cl | 121 - 122 |
| 7 | Cl | $CH_3$ | 3-F | 109 - 112 |
| 8 | Cl | $CH_3$ | 4-F | 125 - 128 |
| 9 | Cl | $CH_3$ | 3-Cl | 131 - 132 |
| 10 | Cl | $CH_3$ | 3-Br | 128 - 131 |
| 11 | Cl | $CH_3$ | $2-CH_3$ | 155 - 157 |
| 12 | Cl | $CH_3$ | $3-CH_3$ | 143 - 144 |
| 13 | Cl | $CH_3$ | $4-CH_3$ | 135 - 138 |
| 14 | Cl | $CH_3$ | $3-C_2H_5$ | 116 - 117 |
| 15 | Cl | $CH_3$ | $3-CF_3$ | 93 - 94 |
| 16 | Cl | $CH_3$ | $3-CH_3O-$ | 141 - 142 |
| 17 | Cl | $CH_3$ | $4-CH_3O-$ | 127 - 128 |
| 18 | Cl | $CH_3$ | $3-iso-C_3H_7O-$ | 103 - 105 |
| 19 | Cl | $CH_3$ | $2,3-(CH_3)_2$ | 191 - 193 |

**Ansprüche**

1. 2-Pyridyloxyacetanilid-Verbindungen der allgemeinen Formel (I)

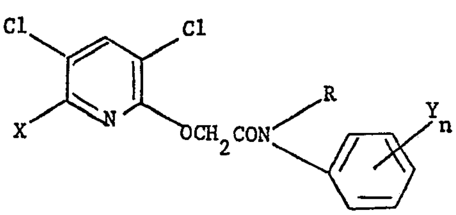

(I)

in der X für ein Wasserstoff-Atom, ein Halogen-Atom, eine $C_1$- bis $C_4$-Alkyl-Gruppe oder eine $C_1$- bis $C_4$-Halogenalkyl-Gruppe, R für eine $C_1$- bis $C_4$-Alkyl-Gruppe, Y für ein Halogen-Atom, eine $C_1$- bis $C_4$-Alkyl-Gruppe, eine $C_1$- bis $C_4$-Alkoxy-Gruppe oder eine $C_1$- bis $C_4$-Halogenalkyl-Gruppe und n für 0, 1, 2 oder 3 steht.

2. 3,5-Dichloro-2-pyridyloxyaceto-N-methylanilid der Formel (1)

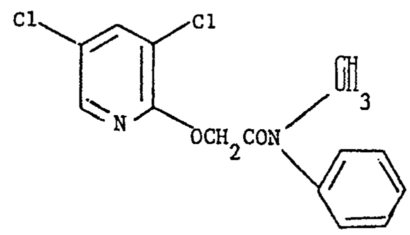

(1)

3. 3,5,6-Trichloro-2-pyridyloxyaceto-N-methylanilid der Formel (3)

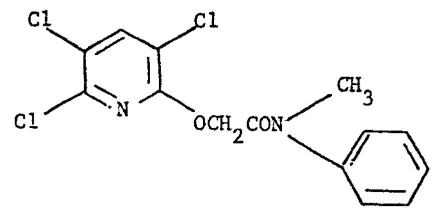

(3)

4. 3,5-Dichloro-6-methyl-2-pyridyloxyaceto-N-methylanilid der Formel (2)

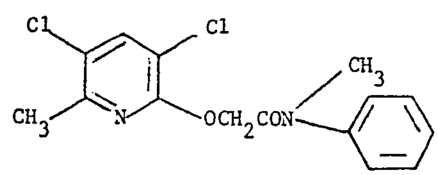

(2)

5. Verfahren zur Herstellung von 2-Pyridyloxyacetanilid-Verbindungen der allgemeinen Formel (I)

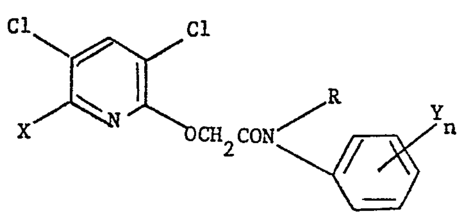

(I)

in der X, R, Y und n die nachstehende Bedeutung haben, dadurch gekennzeichnet, daß ein 2-Chloropyridin der allgemeinen Formel (II)

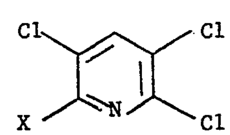

(II)

in der X für ein Wasserstoff-Atom, ein Halogen-Atom, eine $C_1$-$C_4$-Alkylgruppe oder eine $C_1$-$C_4$-Halogenalkyl-Gruppe steht, mit einem Glykolsäure-N-alkylanilid der allgemeinen Formel (III)

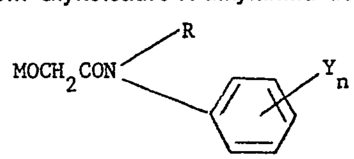

(III)

in der R für eine $C_1$-$C_4$-Alkyl-Gruppe, Y für ein Halogen-Atom, eine $C_1$-$C_4$-Alkoxy-Gruppe oder eine $C_1$-$C_4$-Halogenalkyl-Gruppe, n für 0, 1, 2 oder 3, und M für ein Wasserstoff-Atom oder ein Alkalimetall-Atom steht, wahlweise in Gegenwart eines säurebindenden Mittels, umgesetzt wird.

6. Verfahren zur Herstellung von 2-Pyridyloxyacetanilid-Verbindung der allgemeinen Formel (I)

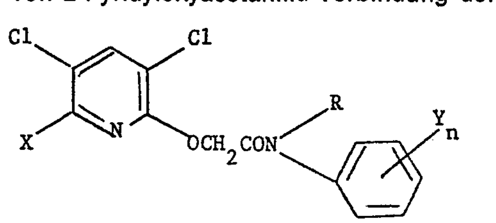

(I)

in der X, R, Y und n die nachstehende Bedeutung haben, dadurch gekennzeichnet, daß ein 2-Pyridinol-Natriumsalz der allgemeinen Formel (IV)

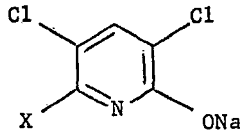

(IV)

in der X für ein Wasserstoff-Atom, ein Halogen-Atom, eine $C_1$-$C_4$-Alkyl-Gruppe oder eine $C_1$-$C_4$-Halogenalkyl-Gruppe steht, mit einem Chloroaceto-N-alkylanilid der allgemeinen Formel (V)

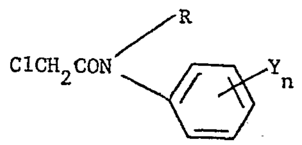

(V)

in der R für eine $C_1$-$C_4$-Alkyl-Gruppe, Y für ein Halogen-Atom, eine $C_1$-$C_4$-Alkyl-Gruppe, eine $C_1$-$C_4$-Alkoxy-Gruppe oder eine $C_1$-$C_4$-Halogenalkyl-Gruppe und n für 0, 1, 2 oder 3 steht, umgesetzt wird.

7. Herbizide Mittel, gekennzeichnet durch einen Gehalt an mindestens einem 2-Pyridyloxyacetanilid der allgemeinen Formel (I) gemäß Anspruch 1.

8. Verfahren zur Bekämpfung von Unkräutern, dadurch gekennzeichnet, daß man 2-Pyridyloxyacetanilide der allgemeinen Formel (I) gemäß Anspruch 1 auf die Unkräuter oder ihren Lebensraum einwirken läßt.

9. Verwendung von 2-Pyridyloxyacetaniliden der allgemeinen Formel (I) gemäß Anspruch 1 zur Bekämpfung von Unkräutern.

10. Verfahren zur Herstellung von herbiziden Mitteln, dadurch gekennzeichnet, daß man 2-Pyridyloxyacetanilide der allgemeinen Formel (I) gemäß Anspruch 1 mit Streckmitteln und/oder oberflächenaktiven Mitteln vermischt.

**Claims**

1. 2-Pyridyloxyacetanilide compounds of the general formula (I)

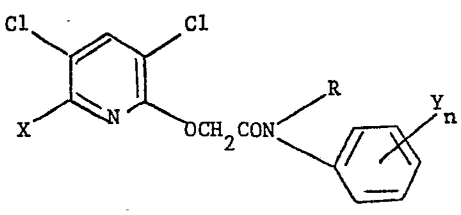

(I)

in which X represents a hydrogen atom, a halogen atom, a $C_1$- to $C_4$-alkyl group or a $C_1$- to $C_4$-halogenoalkyl group, R represents a $C_1$- to $C_4$-alkyl group, Y represents a halogen atom, a $C_1$- to $C_4$-alkyl group, a $C_1$- to $C_4$-alkoxy group or a $C_1$- to $C_4$-halogenoalkyl group and n represents 0, 1, 2 or 3.

2. 3,5-Dichloro-2-pyridyloxyaceto-N-methylanilide of the formula (1)

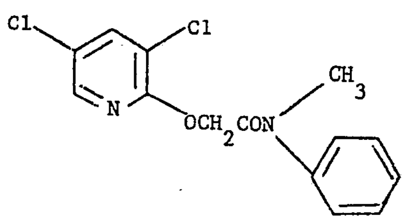

(1)

3. 3,5,6-Trichloro-2-pyridyloxyaceto-N-methylanilide of the formula (3)

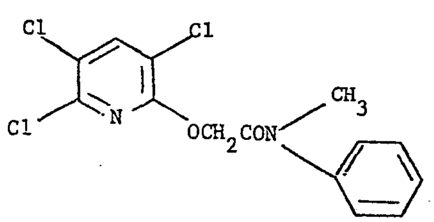

(3)

4. 3,5-Dichloro-6-methyl-2-pyridyloxyaceto-N-methylanilide of the formula (2)

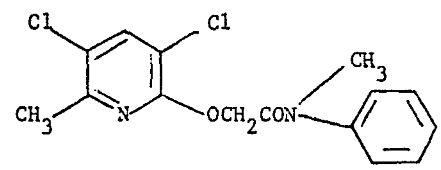

(2)

5. Process for the preparation of 2-pyridyloxyacetanilide compounds of the general formula (I)

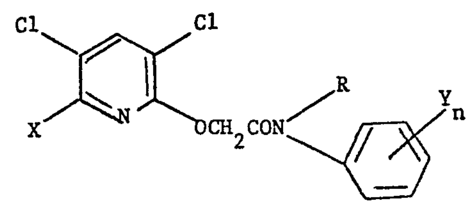

(I)

in which X, R, Y and n have the meaning given below, characterised in that a 2-chloropyridine of the general formula (II)

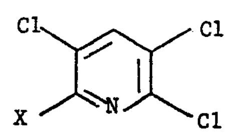

(II)

in which X represents a hydrogen atom, a halogen atom, a $C_1$-$C_4$-alkyl group or a $C_1$-$C_4$-halogenoalkyl group, is reacted with a glycolic acid N-alkylanilide of the general formula (III)

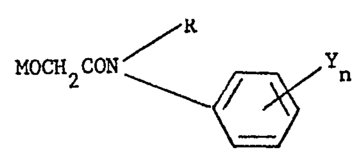

(III)

in which R represents a $C_1$-$C_4$-alkyl group, Y represents a halogen atom, a $C_1$-$C_4$-alkoxy group or a $C_1$-$C_4$-halogenoalkyl group, n represents 0, 1, 2 or 3, and M represents a hydrogen atom or an alkali metal atom, optionally in the presence of an acid-binding agent.

6. Process for the preparation of the 2-pyridyloxyacetanilide compound of the general formula (I)

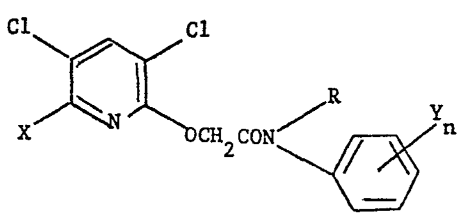

(I)

in which X, R, Y and n have the meaning given below, characterised in that a 2-pyridinol sodium salt of the general formula (IV)

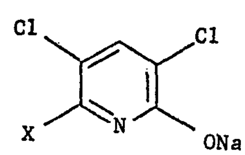

(IV)

in which X represents a hydrogen atom, a halogen atom, a $C_1$-$C_4$-alkyl group or a $C_1$-$C_4$-halogenoalkyl group, is reacted with a chloroaceto-N-alkylanilide of the general formula (V)

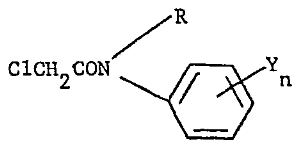

(V)

in which R represents a $C_1$-$C_4$-alkyl group, Y represents a halogen atom, a $C_1$-$C_4$-alkyl group, a $C_1$-$C_4$-alkoxy group or a $C_1$-$C_4$-halogenoalkyl group and n represents 0, 1, 2 or 3.

7. Herbicidal agents, characterised in that they contain at least one 2-pyridyloxyacetanilide of the general formula (I) according to Claim 1.

8. A method of combating weeds, characterised in that 2-pyridyloxyacetanilides of the general formula (I) according to Claim 1 is allowed to act on the weeds or their environment.

9. Use of 2-pyridyloxyacetanilides of the general formula (I) according to Claim 1 for combating weeds.

10. Process for the preparation of herbicidal agents, characterised in that 2-pyridyloxyacetanilides of the general formula (I) according to Claim 1 are mixed with extenders and/or surface-active agents.

**Revendications**

1. Dérivés de 2-pyridyloxyacétanilide de formule générale (I)

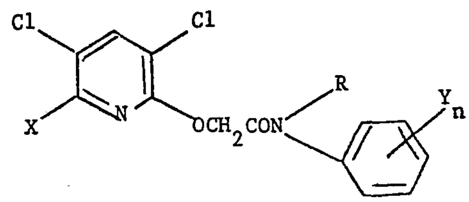

(I)

dans laquelle X représente un atome d'hydrogène, un atome d'halogène, un groupe alkyle en $C_1$-$C_4$ ou un groupe halogénoalkyle en $C_1$-$C_4$, R représente un groupe alkyle en $C_1$-$C_4$, Y un atome d'halogène, un groupe alkyle en $C_1$-$C_4$, un groupe alcoxy en $C_1$-$C_4$ ou un groupe halogénoalkyle en $C_1$-$C_4$ et n est égal à 0, 1, 2 ou 3.

2. 3,5-Dichloro-2-pyridyloxyacéto-N-méthylanilide de formule (1)

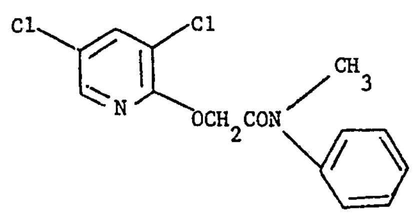

(1)

3. 3,5,6-Trichloro-2-pyridyloxyacéto-N-méthylanilide de formule (3)

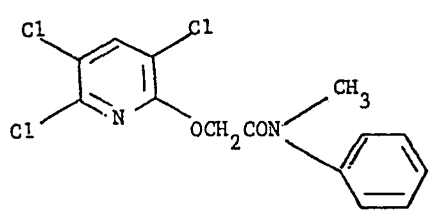

(3)

4. 3,5-Dichloro-6-méthyl-2-pyridyloxyacéto-N-méthylanilide de formule (2)

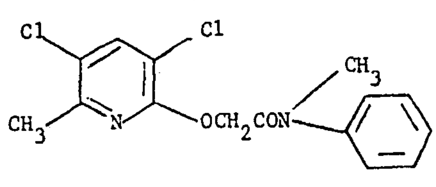

(2)

5. Procédé de préparation des dérivés du 2-pyridyloxyacétanilide de formule générale (I)

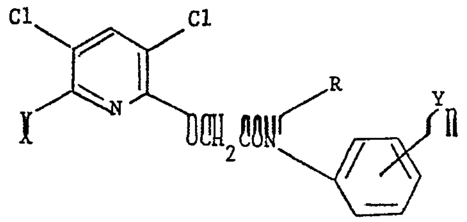

(I)

dans laquelle X, R, Y et n ont les significations indiquées ci-après, caractérisé en ce que l'on fait réagir une 2-chloropyridine de formule générale (II)

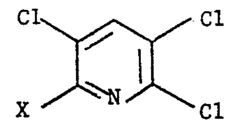

(II)

dans laquelle X représente un atome d'hydrogène, un atome d'halogène, un groupe alkyle en $C_1$-$C_4$ ou halogénoalkyle en $C_1$-$C_4$, avec un N-alkylanilide de l'acide glycolique de formule générale (III)

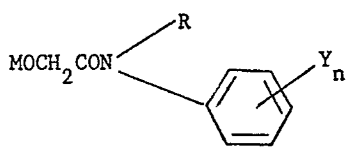

(III)

dans laquelle R représente un groupe alkyle en $C_1$-$C_4$, Y un atome d'halogène, un groupe alcoxy en $C_1$-$C_4$ ou un groupe halogénoalkyle en $C_1$-$C_4$, n est égal à 0, 1, 2 ou 3 et M représente un atome d'hydrogène ou de métal alcalin, si on le désire en présence d'un agent fixant les acides.

6. Procédé de préparation du dérivé du 2-pyridyloxyacétanilide de formule générale (I)

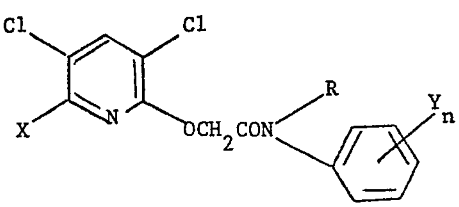

(I)

dans laquelle X, R, Y et n ont les significations indiquées ci-après, caractérisé en ce que l'on fait réagir un sel de sodium de 2-pyridinol de formule générale (IV)

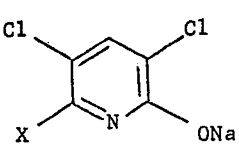

(IV)

dans laquelle X représente un atome d'hydrogène, un atome d'halogène, un groupe alkyle en $C_1$-$C_4$ ou halogénoalkyle en $C_1$-$C_4$, avec un chloracéto-N-alkylanilide de formule générale (V)

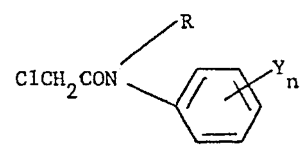

(V)

dans laquelle R représente un groupe alkyle en $C_1$-$C_4$, Y représente un atome d'halogène, un groupe alkyle en $C_1$-$C_4$, un groupe alcoxy en $C_1$-$C_4$ ou halogénoalkyle en $C_1$-$C_4$ et n est égal à 0, 1, 2 ou 3.

7. Produit herbicide, caractérisé en ce qu'il contient au moins un 2-pyridyloxyacétanilide de formule générale (I) selon la revendication 1.

8. Procédé pour combattre les mauvaises herbes, caractérisé en ce que l'on fait agir les 2-pyridyloxyacétanilides de formule générale (I) selon la revendication 1, sur les mauvaises herbes ou leur habitat.

9. Utilisation des 2-pyridyloxyacétanilides de formule générale (I) selon la revendication 1, dans la lutte contre les mauvaises herbes.

10. Procédé de préparation de produits herbicides, caractérisé en ce que l'on mélange les 2-pyridyloxyacétanilides de formule générale (I) selon la revendication 1, avec des diluants et/ou des agents tensioactifs.